# EUROPEAN PATENT SPECIFICATION

(11) **EP 2 585 164 B1**
(45) Date of publication and mention of the grant of the patent: **17.02.2021**
(21) Application number: 11733999.4
(22) Date of filing: 22.06.2011
(51) Int. Cl.: A61M 3/02, F16L 39/00, A61M 39/10

(54) **CONNECTING SYSTEM FOR A PIPE WITH TWO LUMINA**
VERBINDUNGSSYSTEM FÜR EIN ROHR MIT ZWEI LUMINA
SYSTÈME DE RACCORDEMENT POUR TUYAU À DEUX LUMIÈRES

(30) Priority: 23.06.2010 FR 1002638
(43) Date of publication of application: 01.05.2013
(73) Proprietor: B. Braun Medical Sas, 92210 Saint-Cloud (FR)
(72) Inventor: CHEVEREAU, Lionel, 61110 Condeau (FR); COLLIN, Rémi, 61340 Saint-Hilaire-sur-Erre (FR)
(74) Representative: August Debouzy
(86) International application number: PCT/EP2011/003089
(87) International publication number: WO 2011/160834

(56) References cited:
- EP-A2- 0 082 950
- WO-A1-2009/081178
- US-A- 5 681 063
- US-A1- 2002 160 640
- US-A1- 2003 028 182

## Description

The present invention relates to a connecting system for a pipe with two lumina.

When several different fluids have to flow simultaneously in the same pipe, this pipe must have several lumina. Connection devices for pipes with several coaxial lumina are e.g. known from US 2003/028182 A1, WO 2009/081178 A1, US 5,681,063 A, EP 0 082 950 A2 and US 2002/0160640 A1. Different applications can particularly require the simultaneous passage of a liquid, such as water, and a gas, such as air, through the same pipe. For this purpose, a pipe could be used, which has a first lumen situated along an axis and being limited by a cylindrical wall, serving to conduct water, the wall having a second lumen, cylindrical or non-cylindrical, which is used for the passage of air.

This type of pipe makes it difficult to connect two sections of the pipe, or to connect an inlet section of the pipe to a device, particularly in the case where the connection has to be established without taking into account the angular orientation of the two parts, one in relation to the other, a round the axis of the pipe.

In the context of medical care, a pipe provided with two lumina is sometimes used, for example, with an anal irrigation probe. For an anal probe, an inflatable balloon must first of all be filled with air in order to position the probe in the rectum and to keep it there. The probe then allows the instillation of water for washing the rectum. A tank allows the pipe to be supplied with water and a pump with compressed air, but the probe itself is for single use, and a new probe must be connected to the pipe for each new session of colon cleansing. A quick and simple connecting device is therefore advantageous, and this all the more so since these probes are often used by paraplegic or tetraplegic patients, whose dexterity is limited. It is therefore desirable to be able to connect the probe to the end of the inlet pipe with maximum ease, without having to take into account the angular orientation of the said pipe in relation to the probe.

The objective of the present invention is to create a connecting system for a pipe with two lumina, allowing simple connection without taking into account the angular orientation of the parts to connect.

The objective is achieved in accordance with the invention by a connecting system according to claim 1. The present disclosure relates to a connecting system in two parts, for a pipe with a first lumen in the axis of the pipe, and a second lumen outside of the axis of the pipe, the first part having two coaxial cones as connecting elements, the first lumen leading to the first internal cone, and the second lumen leading to the space present between the first cone and the second cone. The second part of the connecting system has two coaxial cones as connecting elements, which are complementary to the two cones of the first part, the two cones of the first part co-operating in a water and/or airtight manner with the two cones of the second part, in order to establish a water and/or airtight continuity between the two lumina of the first and second part, respectively.

The connecting system according to the invention is composed of two parts, of which one can be connected to a pipe with two lumina, and the other can either also be connected to a pipe with two lumina, or to a device such as an anal probe, which functions by means of two different fluids, air and water, for example. The first part of the connecting system has an internal, conical connecting element. The second part of the connecting system has a complementary cone, which, when the system is connected, co-operates in such a way with the cone of the first part that a water and/or airtight effect is obtained. The first lumen of the pipe, which follows the axis of the latter, leads to the internal cone of the first part of the connecting system.

The first part of the connecting system furthermore has an external cone positioned in a concentric manner in relation to the first, internal cone. A space therefore emerges between the internal and external cone, to which leads the second lumen of the pipe, which is situated outside of the axis. The second part of the connecting system has a cone which is complementary to the second cone of the first part, which, when it is connected, co-operates in such a way with the cone of the first part that a water and/or airtight effect is obtained. The external cones of the two parts of the connecting system thus close in a water and/or airtight manner the space between the internal and external cones, so that the fluid conducted through the second lumen of the pipe can thus progress into the corresponding opening of the other piece without loss or leak.

The cones of the first part of the connecting system can have a diameter which decreases in the direction of the second part, in such a way that they can be inserted into the respective complementary cones of the second part during connection. The inverse embodiment is also possible, according to which, the cones of the second part can be inserted into the cones of the first part. Clearly, a cone of one of the parts can also be inserted into the complementary cone of the second part, and the second cone can incorporate its complementary cone.

Preferably, at least one of two complementary connecting elements is made of elastically deformable material. The elastically deformable connecting element can thus be slightly deformed, in particular slightly expanded or squeezed when the complementary connecting element is connected, and the water and/or airtight effect is enhanced. This can for example be realized by using an elastically deformable material for one of the two parts of the connecting system, whereas the other part can be made of rigid material. Of course, also both parts can be made of elastically deformable material, or any other combination is possible with at least one of two complementary connecting elements being elastically deformable.

In the case of connection to a pipe, the first part of the connecting device can have two other coaxial connecting elements, of which at least one has a conical shape. For example, if the internal connection element is conical with increasing diameter which, and if the external connection element is cylindrical, the pipe can be slid against the internal conical connection element. The pipe is thus slightly widened by the cone, that is to say, its internal surface is applied against the internal cone, and thus its internal diameter is slightly increased, and a water and/or airtight effect is obtained. Also, the widening of the internal diameter of the pipe leads to pressure of its external wall on the external cylindrical connecting element, so that the connection becomes fully water and/or airtight. The first lumen of the pipe leads to the internal cone, which communicates with the internal connecting cone of the second part of the connecting system. The second lumen of the pipe, which is situated in the wall of the pipe, leads to a space situated between the two connecting elements.

It is possible to improve the water and/or airtight effect and the fastening of the pipe by designing the two additional connecting elements in a conical manner, the internal connecting element having an increasing diameter and the external connecting element having a decreasing diameter. The wall of the pipe can thus be firmly wedged between the two connecting elements.

The internal connecting elements of the first part can preferably be maintained in the external connecting elements in a coaxial manner by means of connecting struts. Consequently, there is a space between the two cones, guaranteeing that the medium coming from the second lumen can run freely through the first part of the connecting device.

The connection of the first and second parts of the connecting system is preferably strengthened by a snap connection when the parts are connected.

The invention is better understood by means of the attached drawings presented below:
- **Figure 1**: shows an embodiment of the connecting system according to the invention in the non-connected position;
- **Figure 2**: shows the embodiment of Figure 1 in the connected position.

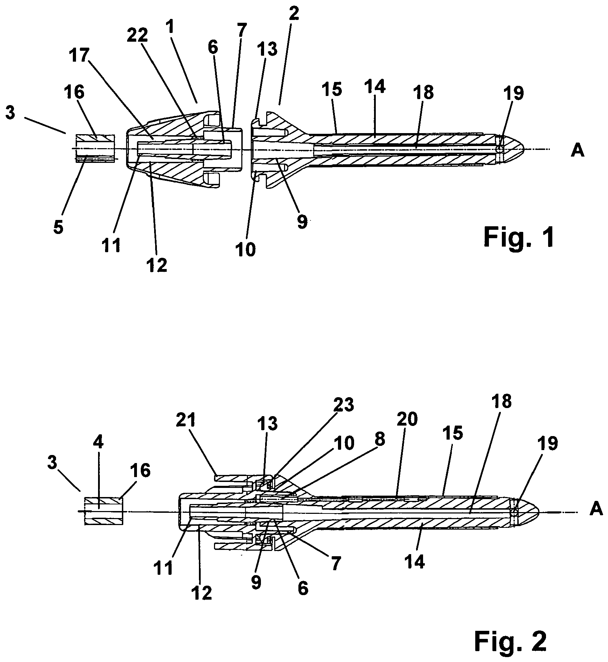

**Figure 1** shows an anal probe **14** with a supply system for water for intestinal cleansing and with an entry system for air to inflate the balloons **15** disposed at the level of the probe. The water supply operates along the axis **A** of the probe, the balloons **15** are divided in segments around the probe **14;** they are shown non-inflated on the Figure and are adjacent to the probe. Consequently, the air inlet operates in an off-central manner. A pipe **3** is used to bring water, which pipe has a first cylindrical lumen **4** along its axis **A** which is limited by a wall **16.** The wall **16** of the pipe **3** has a second lumen **5** serving for the passage of air.

The pipe **3** is connected to the first part **1** of a connecting device in accordance with the invention, which consists of two parts **1, 2.** The second part **2** of the connecting device is directly linked to the anal probe **14.** However, the connecting device **1, 2** could alternatively be linked to other equipment or to another pipe, for example for creating an extension.

The first part **1** of the connecting device, on the connecting side towards the second part **2,** has two coaxial cones **6, 7.** The first lumen **4** of the pipe **3** of the water supply system leads to the internal cone **6.** The second lumen **5** of the air supply system leads to the space **8** situated between the internal cone **6** and the external cone **7.**

The second part **2** of the connecting device has two coaxial cones **9, 10,** which are complementary to the cones **6, 7** of the first part. The dimensions of the cones are such that, in order to connect the two parts of the connecting device, the two cones of the first part co-operate in a water and airtight manner with the two cones of the second part. The second part 2 is made of elastically deformable material, and the dimension of the cones 9, 10 are such that they are slightly expanded when the cones **6, 7** of the first part are inserted into them, and form a water and airtight connection when the device is in the connected position.

In order to connect the pipe **3,** the first part **1** of the connecting device also has two coaxial connecting elements **11, 12,** both having conical shape in the example in this figure. The diameter of the internal cone **11** increases, whereas that of the external cone **12** reduces. The pipe **3** can thus be tightened with the central lumen **4** in a water and airtight manner against the internal cone **11.** The external cone **12** surrounds external wall **16** of the pipe **3** in a water and airtight manner, in such a way that the lumen **5** situated in the wall **16** of the pipe **3** leads to the space **17** between the cones **11, 12.**

Furthermore, it can be seen on Figure 1 that the internal connecting element comprising the two cones **11** and **6** is kept in the external connecting element comprising the cones **12** and **7.** This is preferably achieved by means of connecting struts **22.** The intermediary space **17** between the cones **11** and **12** and the intermediary space **8** between the cones **6** and **7** can thus communicate with one another. The air arriving through the lumen **5** of the pipe **3** into the intermediary space **17** is thus lead into the first part **1** of the connecting system.

**Figure 2** shows the embodiment from Figure 1 in the connected position. It can be seen that the two internal cones **6, 9** are inserted in such a way that the water of the central lumen **4** of the pipe **3** circulates through the lumen **18** of the probe and can flow through the orifices **19.**

The space **8** between the internal cone **6** and the external cone **7** of the first part **1** of the connecting device is closed by the connecting elements **9, 10** of the first part, so that the air which comes out here through the lumen **5** of the pipe **3** is transported by the channel **20** of the probe **14** and inflates the balloon **15** situated on the probe **14.**

The connection of the two parts **1, 2** of the connecting system is strengthened by means of a snap mechanism **13, 14.** Pressure on the push-button **21** allows for the assembly to be released, releasing the teeth **14** in the groove **13** of the second part **2** for separating the two parts **1, 2.**

## Claims

1. Connecting system in two parts (1,2) for a pipe (3) comprising a first lumen (4) in the axis (A) of the pipe, and a second lumen (5) in the wall (16) of the pipe,
the first part (1) having two coaxial cones (6, 7) as connecting elements, the first lumen (4) leading to the first internal cone (6) and the second lumen (5) leading to the space (8) present between the first cone (6) and the second cone (7),
the second part (2) having two coaxial cones (9, 10) as connecting elements, which are complementary to the two cones (6, 7) of the first part (1),
wherein the two cones (6, 7) of the first part (1) co-operating in a water and/or airtight manner with the two cones (9, 10) of the second part (2) in order to connect the two parts (1,2)
**characterized in that**
the first part (1) has an internal connecting element (11) and an external connecting element (12) in order to connect the pipe (3), said internal and external connecting elements (11, 12) being coaxial, **in that** the external connecting element (12) is adapted to surround an external wall (16) of the pipe (3) in a water and airtight manner in such a way that the lumen (5) situated in the wall (16) of the pipe (3) leads to the space (17) between the connecting elements (11, 12), and **in that** at least the internal connecting element (11) has a conical shape.

2. Connecting system according to claim 1, **characterised in that** the cones (6, 7) of the first part (1) can be inserted in a water and/or airtight manner into the two cones (9, 10) of the second part (2).

3. Connecting system according to claim 1, **characterised in that** the cones (9, 10) of the second part (2) can be inserted in a water and/or airtight manner into the two cones (6, 7) of the first part (1).

4. Connection system according to one of claims 1 to 3, **characterized in that** at least one of two complementary connecting elements (6, 9; 7, 10) is made of elastically deformable material.

5. Connecting system according to one of claims 1 to 4, **characterised in that** the internal connecting element (11) has conical shape with increasing diameter.

6. Connecting system according to one of claims 1 to 5, **characterised in that** the external connecting element (12) has conical shape with decreasing diameter.

7. Connecting system according to one of claims 1 to 5, **characterised in that** the external connecting element (12) has a cylindrical shape.

8. Connecting system according to one of claims 1 to 7, **characterised in that** the internal connecting elements (6, 11) of the first part (1) are kept in a coaxial manner in the external connecting elements (7, 12) of the first part by means of connecting struts (22).

9. Connecting system according to one of claims 1 to 8, **characterised in that** the first part (1) and the second part (2) are secured in the connected position by means of a snap connection (13, 14).

## Patentansprüche

1. Zweiteiliges (1, 2) Verbindungssystem für ein Rohr (3), umfassend ein erstes Lumen (4) in der Achse (A) des Rohrs und ein zweites Lumen (5) in der Wand (16) des Rohrs,
wobei der erste Teil (1) zwei koaxiale Kegel (6, 7) als Verbindungselemente aufweist, das erste Lumen (4) zum ersten internen Kegel (6) und das zweite Lumen (5) zum Raum (8) zwischen dem ersten (6) und zweiten Kegel (7) führt,
wobei der zweite Teil (2) zwei koaxiale Kegel (9, 10) als Verbindungselemente aufweist, die die beiden Kegel (6, 7) des ersten Teils (1) ergänzen,
wobei die beiden Kegel (6, 7) des ersten Teils (1) auf wasser- und/oder luftdichte Art mit den beiden Kegeln (9, 10) des zweiten Teils (2) zusammenwirken, um die beiden Teile (1, 2) zu verbinden,
**dadurch gekennzeichnet, dass**
der erste Teil (1) ein internes Verbindungselement (11) und ein externs Verbindungselement (12) aufweist, um das Rohr (3) zu verbinden, wobei das interne und externe Verbindungselement (11, 12) koaxial sind, dass das externe Verbindungselement (12) dazu angepasst ist, um eine Außenwand (16) des Rohrs (3) auf wasser- und luftdichte Art zu umschließen, so dass das in der Wand (16) des Rohrs (3) angeordnete Lumen (5) zum Raum (17) zwischen den Verbindungselementen (11, 12) führt, und dass mindestens das interne Verbindungselement (11) kegelförmig ist.

2. System nach Anspruch 1, **dadurch gekennzeichnet, dass** die Kegel (6, 7) des ersten Teils (1) auf wasser- und/oder luftdichte Art in die beiden Kegel (9, 10) des zweiten Teils (2) eingeführt werden können.

3. System nach Anspruch 1, **dadurch gekennzeichnet, dass** die Kegel (9, 10) des zweiten Teils (2) auf wasser- und/oder luftdichte Art in die beiden Kegel (6, 7) des ersten Teils (1) eingeführt werden können.

4. System nach einem der Ansprüche 1-3, **dadurch gekennzeichnet, dass** mindestens eines der einander ergänzenden Verbindungselemente (6, 9; 7, 10) aus elastisch verformbaren Material besteht.

5. System nach einem der Ansprüche 1-4, **dadurch gekennzeichnet, dass** das interne Verbindungselement (11) kegelförmig ist und dessen Durchmesser zunimmt.

6. System nach einem der Ansprüche 1-5, **dadurch gekennzeichnet, dass** das externe Verbindungselement (12) kegelförmig ist und dessen Durchmesser abnimmt.

7. System nach einem der Ansprüche 1-5, **dadurch gekennzeichnet, dass** das externe Verbindungselement (12) zylindrisch ist.

8. System nach einem der Ansprüche 1-7, **dadurch gekennzeichnet, dass** die internen Verbindungselemente (6, 11) des ersten Teils (1) mittels Verbindungsstreben (22) in den externen Verbindungselementen (7, 12) des ersten Teils koaxial gehalten werden.

9. System nach einem der Ansprüche 1-8, **dadurch gekennzeichnet, dass** der erste Teil (1) und der zweite Teil (2) mittels einer Einrastverbindung (13, 14) in der verbundenen Stellung gesichert werden.

## Revendications

1. Système de raccord à deux pièces (1, 2) destiné à un tuyau (3) comprenant une première lumière (4) dans l'axe (A) du tuyau et une seconde lumière (5) dans la paroi (16) du tuyau,
la première pièce (1) présentant comme éléments de raccord deux cônes coaxiaux (6, 7), la première lumière (4) menant au premier cône interne (6) et la seconde lumière (5) à l'espace (8) présent entre le premier cône (6) et le second cône (7), la seconde pièce (2) présentant comme éléments de raccord deux cônes coaxiaux (9, 10) complémentaires aux deux cônes (6, 7) de la première pièce (1),
les deux cônes (6, 7) de la première pièce (1) coopérant de manière étanche à l'eau et/ou à l'air avec les deux cônes (9, 10) de la seconde pièce (2) afin de raccorder les deux pièces (1, 2)
**caractérisé en ce que**
la première pièce a un élément de raccord interne (11) et un élément de raccord externe (12) pour raccorder le tuyau (3), lesdits éléments de raccord interne et externe (11, 12) étant coaxiaux, **en ce que** l'élément de raccord externe (12) est adapté pour entourer la paroi externe (16) du tuyau (3) de manière étanche à l'eau et à l'air de sorte que la lumière (5) située dans la paroi (16) du tuyau (3) mène à l'espace (17) entre les éléments de raccord (11,12), et **en ce que** au moins l'élément de raccord interne (11) est de forme conique.

2. Système de raccord selon la revendication 1, **caractérisé en ce que** les cônes (6, 7) de la première pièce (1) peuvent être insérés de manière étanche à l'air et /ou à l'eau dans les deux cônes (9, 10) de la seconde pièce (2).

3. Système de raccord selon la revendication 1, **caractérisé en ce que** les cônes (9, 10) de la seconde pièce (2) peuvent être insérés de manière étanche à l'air et/ou à l'eau dans les deux cônes (6, 7) de la première pièce (1).

4. Système de raccord selon l'une des revendications 1 à 3, **caractérisé en ce que** au moins un des deux éléments de raccord complémentaires (6, 9 ; 7, 10) sont fait en matériau élastiquement déformable.

5. Système de raccord selon l'une des revendications 1 à 4, **caractérisé en ce que** l'élément de raccord interne (11) est de forme conique et de diamètre croissant.

6. Système de raccord selon l'une des revendications 1 à 5, **caractérisé en ce que** l'élément de raccord externe (12) est de forme conique et de diamètre décroissant.

7. Système de raccord selon l'une des revendications 1 à 5, **caractérisé en ce que** l'élément de raccord externe (12) est de forme cylindrique.

8. Système de raccord selon l'une des revendications 1 à 7, **caractérisé en ce que** les éléments de raccord internes (6, 11) de la première pièce (1) sont maintenus de manière coaxiale dans les éléments de raccord externes (7, 12) de la première pièce au moyen de traverses de jonction (22).

9. Système de raccord selon l'une des revendications 1 à 8, **caractérisé en ce que** la première pièce (1) et la seconde pièce (2) sont consolidées en position raccordée par l'intermédiaire d'un assemblage par encliquetage (13, 14).
